Europäisches Patentamt

European Patent Office

Office européen des brevets

(11) Publication number: **0 373 722**
**A2**

## EUROPEAN PATENT APPLICATION

(21) Application number: 89203160.0

(22) Date of filing: **11.12.89**

(51) Int. Cl.⁵: **A61N 2/12**

(30) Priority: **12.12.88 JP 160438/88**
**19.07.89 JP 188432/89**

(43) Date of publication of application:
**20.06.90 Bulletin 90/25**

(84) Designated Contracting States:
**AT BE CH DE ES FR GB GR IT LI LU NL SE**

(71) Applicant: **SHIN-ATSU-SHIN CLINIC, INC.**
**Yutaka Bldg., No. 1-624 Ikebukuro**
**Toshima-ku Tokyo(JP)**

(72) Inventor: **Nakagawa, Hajime**
**Jukou Maison, No. 4-457 Ikebukuru**
**Toshima-ku, Tokyo(JP)**

(74) Representative: **de Bruijn, Leendert C. et al**
**Nederlandsch Octrooibureau**
**Scheveningseweg 82 P.O. Box 29720**
**NL-2502 LS 's-Gravenhage(NL)**

(54) **Rotary magnetic therapeutical appliance.**

(57) A rotary magnetic therapeutical appliance for giving magnetism to a therapeutical point. The appliance has a magnet positioned close to a therapeutical point and a rotary mechanism for rotating the magnet. Different magnetic poles are alternately arrayed in a peripheral direction on a magnet front end surface. A head guide incorporating the magnet is provided with an impingement member impinging on a skin face. The impingement member is formed, on its front surface, with acute stimulative projections.

FIG. 1

EP 0 373 722 A2

# ROTARY MAGNETIC THERAPEUTICAL APPLIANCE

## BACKGROUND OF THE INVENTION

The present invention relates to a rotary magnetic therapeutical appliance intended to enhance medical effects by giving stimuli of magnetic fluctuations to a diseased part, e.g., keiketsu (parts effective for acupuncture), keiraku (a circulatory and reactive system which links keiketsu one another) and the like of a medical treatment receiving body conceived as a person who undergoes a medical treatment.

There have heretofore been practiced a variety of physical therapeutical methods of performing a medical treatment based on natural curing functions by exciting or restraining nerves while giving physical stimuli to so-called tsubo (vital parts) such as keiketsu, keiraku and the like which exist in a human body.

One of these methods is arranged to obtain medical effects by imparting magnetism to specific parts, i.e., the vital parts including keiketsu, keiraku and the like of the human body. For instance, a prior art magnetic therapeutical appliance is disclosed in Japanese Utility Model Laid-Open Publication No.60-20247, the arrangement being such that a permanent magnet fixed to a top end of a vibrator is vibrated up and down, and there are generated fluctuant magnetic fields whose sparseness and denseness are distributed in a vertical direction to a therapeutical point. Furthermore, a rotary magnet massager is disclosed in Japanese Utility Model Laid-Open Publication No.60-106641, wherein a magnet member is moved on a circular orbit about the therapeutical point.

In these conventional magnetic therapeutical appliances, however, as in the first case, the magnetic stimuli are nothing but intermittent stimuli of the same poles of N or S even when being combined with mechanical up-and-down vibrations with respect to the therapeutical point defined as a diseased part or the like of a human body. Sufficient medical effects can not be obtained. Fluctuations due to the movement of the same polar plane with respect to action plane, as in the second case, imply mere intermittent stimuli of the same poles of N or S with respect to the action plane. In this case, similarly the sufficient medical effects can not be attained.

## SUMMARY OF THE INVENTION

It is a primary object of the present invention, which is devised under the aforementioned circumstances incidental to the prior art, to provide a rotary magnetic therapeutical appliance capable of exhibiting sufficient medical effects by alternately fluctuating magnetic planes of different poles and imparting the fluctuation to a skin face and the like of a diseased part, keiketsu and keiraku of a treated person.

To accomplish the foregoing object, according to one aspect of the invention, there is provided a rotary magnetic therapeutical appliance comprising a magnet having its front end surface disposed in close proximity to a therapeutical point of the treated person, and a rotary mechanism for rotating the magnet with the center of the front end surface serving as a center of an axial core, characterized in that a proper number of different magnetic poles are alternately arrayed in a peripheral direction on the front end surface of the magnet.

The magnet is incorporated in a head guide having its front end surface provided with an impingement member which impinges on the skin face. A front surface of the impingement member is formed with a multiplicity of stimulative projections each having a substantially acute apex. The head guide, as the case may be, is movable to and fro.

The stimulative projections each assuming a substantially quadrangular pyramidal shape can be arrayed on the front surface of the impingement member.

In the rotary magnetic therapeutical appliance according to the present invention, the magnet is rotated by operating the rotary mechanism. When a top end of the head guide is pushed against a therapeutical point of a human body, the magnet approaches the therapeutical point.

Upon rotating the magnet in close proximity to the therapeutical point, the different magnetic polar planes on the front end surface thereof alternately give magnetic stimuli to keiketsu, keiraku and the like about the therapeutical point, thereby exciting or restraining the nerves associated therewith. As a result, the nerve activities are brought into a sound state, thus presenting medical effects of activating the natural curing functions.

At this time, the impingement member at the front end of the head guide which is pushed against the therapeutical point gives pushing stimuli to the skin face of the diseased part by means of the stimulative projections, thus massaging the skin face.

## BRIEF DESCRIPTION OF THE DRAWINGS

Other objects and advantages of the invention will become apparent during the following discussion taken in conjunction with the accompanying drawings, in which:

The Figures in combination show one embodiment of the present invention.

FIG. 1 is a partially cut-away side view;

FIG. 2 is a front elevation depicting an impingement member;

FIG. 3 is a partially cut-away side view illustrating a rotary magnet member; and

FIG. 4 is a front elevation depicting a magnet.

## DESCRIPTION OF THE PREFERRED EMBODIMENT

One embodiment of the present invention will hereinafter be described with reference to the accompanying drawings.

Designated at 1 in the Figures is a bottomed substantially cylindrical case which encases a vibrating mechanism 10. The case 1 is fitted with a handle 2, which is in turn mounted with a switch connected to a predetermined power source. Driving of the vibrating mechanism 10 is controlled by this switch.

Fixed to an opening portion formed in the front of the case 1 is a front cover 3 to which a head guide retaining member 4 formed in a substantially cylindrical shape is fixed. The head guide retaining member 4 accommodates a head guide 5 so retained by this member 4 as to advanceable and retreatable.

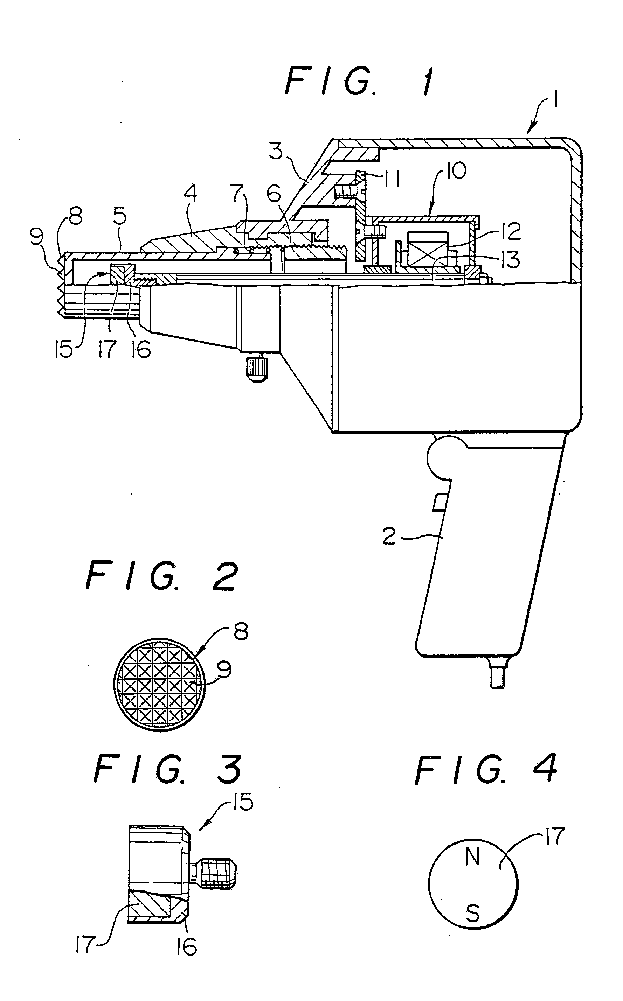

The head guide advances and retreats with the help of a resilient force of a coil spring 7 which is, as depicted in FIG. 1, contractibly interposed between a front end face of a fixing cylindrical member 6 fixed to a rear portion of the head guide retaining member 4 and a protruded portion shaped on a rear periphery of the head guide 5. When pushing backwards a top end of the head guide 5, the head guide 5 itself moves back resisting the resilient force of the spring 7. Upon a release of the pushing force, the head guide 5 returns to a front position.

As discussed above, the head guide 5 is advanceable and retreatable, whereby magnitudes of magnetic fields given from a magnet 17 which will hereinafter be mentioned can arbitrarily be adjusted.

A top end of the head guide 5 is closed, and a closed front end surface is fitted with an impingement member 8 which impinges on a skin face of a diseased part of a person who undergoes a medical treatment. Arrayed on a front surface of the impingement member 8 are a multiplicity of stimulative projections 9 each assuming a substantially quadrangular pyramidal configuration to have a substantially acute apex (see FIGS 1 and 2).

Note that the impingement member 8 is integrally formed of a synthetic resin or the like by, e.g., extrusion molding. The material thereof is not, however, limited to the synthetic resin, and the front surface of the impingement member 8 is formed with as many stimulative projections 9 as possible. The illustrated stimulative projection is formed to assume the substantially quadrangular pyramidal shape. The configuration thereof may, however, be modified with other conical or pyramidal shapes, for instance, conical or polygonal pyramidal configurations. The apexes are, as the case may be, slightly round. A mode of arrangement is arbitrary, wherein the stimulative projections 9 are not necessarily arrayed in vertical and crosswise directions. In short, each apex of the multiplicity of stimulative projections 9 is substantially acute to provide pointwise stimulating effects on the skin of the treated person.

The rotary mechanism 10 is fixedly disposed on a rear surface of a fixed base 11 fixed to a rear portion of the front cover 3. As depicted in the Figure, a motor shaft 13 of a motor 12 secured to the fixed base 11 is adapted to be rotatable within the head guide 5 (see FIG. 1).

Fastened via a screw or other fixing means to a top end of the thus rotated motor shaft 13 is a rotary magnet member 15, as illustrated in FIG. 3, having its rear surface from which an external thread is protruded and formed, at a front portion thereof, with a magnet holder 16 including a recessed circular accommodation part. The accommodation part accommodates a magnet 17 including its front end surface formed with a magnetic plane on which a single piece or a plurality of magnetic poles are alternately arrayed in a peripheral direction.

Different polarity segments are arranged on the same magnetic plane of the magnet 17. For instance, as illustrated in FIG. 4, one semi-circular segment is an N pole, whereas the other semi-circular segment is an S pole. If a plurality of combinations of the N and S poles are provided, the polar segments are radially alternately arrayed in the peripheral direction. Differences between these polarities can also be provided, without being confined to integral formation on the front end surface of the magnet 17, by disposing the radial magnetic segments, one end surface of which is the N or S pole, on the disc so as to be alternately adjacent to each other.

A capacity of the magnet 17 is set to, e.g.,

2,000 gausses or more so that a therapeutical point of a human body is supplied with a magnetic field from the rear of the impingement member 8 disposed at the front end of the head guide 5; and the magnet 17 is arranged to act on the human body with 10 ~ 20 gausses.

The magnet 17 is also arranged to steer clear of the rear surface of the front end closed portion of the head guide 5 which is to move back, and the rotation thereof is not hindered even when the head guide 5 is in its retreat position.

Next, one example of utilization of the rotary magnetic therapeutical appliance will hereinafter be described. When the rotary mechanism 10 is actuated by turning ON the power source, the rotary magnet member 15 provided at the top end is rotated through the motor shaft 13. The top end of the head guide 5 is pushed against the therapeutical point of the human body, and the head guide 5 is thereby retreated, at which time the rotary magnet member 15 comes close to the therapeutical point.

Upon rotating the rotary magnet member 15 is close proximity to the therapeutical point, the different magnetic polar planes, viz., the N and S poles on the front end surface thereof serve to magnetically stimulate the diseased part and also various vital parts such as keiketsu, keiraku and the like in the diseased part. The nerves pertaining thereto are thus excited or restrained, thereby making the nerve activities sound. In this manner, there are given medical effects of activating the natural curing functions.

In this case, the impingement member 8 at the front end of the head guide 5 which is pushed against the therapeutical point works to give the pushing stimuli to the skin face of the diseased part with the stimulative projections 9 thereof.

As discussed above, according to the present invention having the foregoing construction, the fluctuant magnetic fields where the polarities alternately differ are imparted to the therapeutical point on the skin face, which may include the diseased part, keiketsu and keiraku of the treated person. As a result, multiple cells associated therewith are activated. The nerves and other related parts are excited or restrained to recover the natural healing power, thus enhancing the medical effects.

Namely, this is attributed to the arrangement that the rotary magnetic therapeutical appliance of the invention has the magnet 17 including its front end surface disposed in the vicinity of the therapeutical point of the treated person, the magnet 17 is rotated by the rotary mechanism 10 with the center of its front end surface serving as a center of the axial core, and a proper number of different magnetic poles are alternately arrayed in the peripheral direction on the front end surface of the magnet 17 . With this arrangement, there are given the stimuli of the magnetic fluctuations in which the different polarities act alternately on the diseased part, e.g., keiketsu, keiraku and the like, thereby increasing the medical effects.

Besides, the impingement member 8 impinging on the skin face is provided on the front end surface of the head guide 5 incorporating the magnet 17. The front surface of the impingement member 8 is formed with the multiplicity of stimulative projections 9 each having the substantially acute apex. Based on this construction, the stimulative projections 9 are pushed against the skin face, an the same effects as those in the conventional moxibustion and acupuncture by use of needles can thereby the obtained.

The stimulative projections 9 each assuming the substantially quadrangular pyramidal shape are arrayed on the front surface of the impingement member 8, and hence the secure massaging action on the skin face is exhibited. Since it is not required that the human body is stuck inwards with the needle, the therapeutical appliance of the invention is safety in a sanitary sense and further eliminates a probability that the treated person is infected with various kinds of diseases.

Although the illustrative embodiment of the present invention has been described in detail with reference with the accompanying drawings, it is to be understood that the invention is not limited to that precise embodiment. Various changes or modifications may be effected therein by one skilled in the art without departing from the scope or spirit of the invention.

## Claims

1. A rotary magnetic therapeutical appliance for giving magnetism to a therapeutical point of a person who undergoes a medical treatment, comprising:
a magnet having its front end surface disposed in close proximity to said therapeutical point of the treated person; and
a rotary mechanism for rotating said magnet with the center of said front end surface serving as a center of an axial core, characterized in that a proper number of different magnetic poles are alternately arrayed in a peripheral direction on said front end surface of said magnet.

2. The rotary magnetic therapeutical appliance as set forth in Claim 1, wherein said magnet is incorporated in a head guide having its front end surface provided with an impingement member which impinges on a skin face, and a front surface of said impingement member is formed with a multiplicity of stimulative projections each having a substantially acute apex.

3. The rotary magnetic therapeutical appliance as set forth in Claim 2, wherein said head guide is arranged to move back and forth.

4. The rotary magnetic therapeutical appliance as set forth in Claim 2, wherein said stimulative projections each assuming a substantially quadrangular pyramidal shape can be arrayed on said front surface of said impingement member.

5. The rotary magnetic therapeutical appliance as set forth in Claim 3, wherein said stimulative projections each assuming a substantially quadrangular pyramidal shape can be arrayed on said front surface of said impingement member.

FIG. 1

FIG. 2

FIG. 3

FIG. 4